# EUROPÄISCHE PATENTSCHRIFT

(11) **EP 0 585 896 B1**
(45) Veröffentlichungstag und Bekanntmachung des Hinweises auf die Patenterteilung: **04.12.1996**
(21) Anmeldenummer: 93113966.1
(22) Anmeldetag: 01.09.1993
(51) Int. Cl.: A61K 31/045

(54) **Arzneimittel zur topischen Anwendung am Auge zur Behandlung des erhöhten intraokularen Drucks**
Medicament for tropical application at the eye for the treatment of increased intraocular pressure
Médicament pour l'application topique à l'oeil pour le traitement de la tension intraoculaire élevée

(30) Priorität: 04.09.1992 DE 4229494
(43) Veröffentlichungstag der Anmeldung: 09.03.1994
(73) Patentinhaber: Basotherm GmbH, D-88396 Biberach (DE)
(72) Erfinder: Franz, Helmut, Dr., D-88400 Biberach 1 (DE); Kompa, Hannes Edgar, D-88400 Biberach 1 (DE); Rozman, Tibor, Prof. Dr., D-88400 Biberach 1 (DE)

(56) Entgegenhaltungen:
- DE-A- 3 119 051
- DE-A- 3 700 379
- DE-A- 3 734 835
- FR-A- 2 661 832
- GB-A- 2 072 015
- US-A- 4 201 706
- ANN. D'OCULIST, Bd. 210, Nr. 1, 1977, Seiten 63-69; M.VIRNO ET AL.: 'Emploi des agents osmotiques et leurs indications en ophthalmologie'

## Beschreibung

Die vorliegende Erfindung betrifft die Verwendung der Polyhydroxyalkohole Sorbit, Mannit, Inosit oder Xylit sowie deren Mischungen zur Herstellung eines Arzneimittels zur topischen Anwendung am Auge zur Behandlung des erhöhten intraokularen Drucks.

Erhöhter intraokularer Druck kann zu einer Schädigung des Sehnervs führen und damit zur Einschränkung des Sehvermögens, was sich in einer langsam zunehmenden Einschränkung des Gesichtsfeldes bemerkbar macht und bei unterlassener Behandlung bis zur Blindheit führen kann. Die Ursache dieser Erkrankung besteht in einem Ungleichgewicht zwischen Kammerwasserproduktion und dem korrespondierenden Drainagesystem.

Die Kammerwasserproduktion erfolgt über das Gefäßsystem der Uvea, insbesondere über das Gefäßsystem der Ciliarkörper und die Belegzellen des Ciliarkörpers. Der Abfluß des Kammerwassers geschieht über das Trabekelwerk. An der Ausbildung des erhöhten intraokularen Drucks sind folgende pathologische Mechanismen beteiligt:
- eine partielle Abflußbehinderung am Trabekelwerk durch Fibrosierung oder durch erhöhten Widerstand der abführenden Gefäße.
- Erhöhter Perfusionsdruck (Filtrationsdruck) der Ciliarge- fäße sowie Flußbehinderung durch erhöhten Widerstand der Venolen führen zu einer gesteigerten Kammerwasserbildung.
- Bruch der Blut-Wasserschranke des uvealen Gefäßsystems mit Plasmaaustritt und Ansteigen des kolloidosmotischen Drucks, in dessen Folge zum Ausgleich mehr Wasser gebildet wird. Störungen der intraokularen Blut-Wasserschranke treten auch häufig nach intraokularen Operationen, z. B. nach Trabekulektomie oder nach Kataractoperationen auf.

Bei der Behandlung des erhöhten intraokularen Drucks mit β-Blockern, die über eine Vasokonstriktion des Gefäßsystems der Uvea eine verminderte Kammerwasserproduktion bewirken, tritt als Nebenwirkung eine negative Beeinflussung des okularen Blutflusses auf (A. Yoshida, G.T. Feke et al., Ophthalmic. Res. 23, 162-170 (1991) und M. Langham, Arvo Meeting 1992, Sarasota). Dieser nachteilige Effekt ist bei der Anwendung osmoregulatorisch wirkender Verbindungen nicht zu erwarten.

Die in der Literatur beschriebene orale oder intravenöse Applikation von hypertonen Substanzen, wie von Harnstoff oder Polyalkoholen, die eine osmotische Wirkung auf die intraokulare Flüssigkeit ausüben, ist zum Teil mit erheblichen systemischen Nebenwirkungen verbunden. P. Segal und J. Smolarz-Dudarewicz beschreiben in Klin. Monatsbl. Augenheilkd. 150, 509-522 (1967) die augendrucksenkende Wirkung von Glycerin, Mannit und Sorbit bei peroraler Verabreichung. Im Fall von Glycerin überwiegen als Nebenwirkungen die auf die osmotische Wirkung des Medikaments auf das zentrale Nervensystem zurückzuführenden Erscheinungen wie Kopfschmerzen, Schläfrigkeit und Schwindelgefühle, während bei der Anwendung von Mannit und Sorbit zusätzlich häufig Durchfälle auftreten.

Bei intravenöser Applikation von Mannit traten neben Schwindel, Kopfschmerz und Schüttelfrost auch Atemnot mit Zyanose und Kaltwerden der Glieder auf, und, besonders bei Patienten mit Nieren- und Herzinsuffizienz, auch Präkordialschmerz mit Veränderungen der EKG-Kurve sowie in Einzelfällen fulminantes Herzversagen. Für die intravenöse Anwendung von Harnstofflösungen werden als Begleiterscheinungen Appetitlosigkeit, Übelkeit, Temperaturanstieg, elektrokardiographische Veränderungen und Lungenödem beschrieben, sodaß eine entsprechende Therapie für Patienten mit Leber-, Nieren- und Herzkreislaufinsuffizienz kontraindiziert ist (vgl. G.B. Bietti, Klin. Monatsbl. Augenheilkd. 150, 317-324 (1967)).

Die bereits 1938 beschriebene Behandlung von erhöhtem intraokularen Druck durch intravenöse Infusion von Sorbitlösungen konnte sich ebenfalls nicht durchsetzen [J. Bellows et al., J. Arch. Ophth. (A.M.A.), 20, 1036-1043 (1938)].

Aus diesen Gründen wäre es wünschenswert, eine Methode zur Behandlung von erhöhtem intraokularen Druck zu entwickeln, bei der durch eine topische Anwendung am Auge eine gezielte und auf den gewünschten Wirkort lokal beschränkte Entwässerung und damit eine Erniedrigung des intraokularen Drucks erreicht werden kann, wodurch die vorstehend erwähnten systemischen Nebenwirkungen osmoregulatorisch wirkender Verbindungen vermieden werden können, und bei der keine negative Beeinflussung des okularen Blutflusses auftritt.

In FR-A-2661832 wird eine topisch anzuwendende ophthalmische Lösung zur Senkung des intraokularen Drucks beschrieben, welche den Dopamin-Rezeptor-Antagonisten Bromocryptine als aktives Wirkprinzip und lediglich als isotonisierendes Mittel unter anderem Sorbit oder Mannit enthalten kann. Es wurde jedoch nicht erkannt, daß Sorbit oder Mannit selbst eine drucksenkende Wirkung besitzt.

In der DE-A-31 19 051 ist eine ophthalmische Lösung zur topischen Behandlung des grünen Stars bzw. der okulären Hypertension, enthaltend den Wirkstoff 2-(3-tert.-Butylamino-2-hydroxypropylthio)-4-(5-carbamoyl-2-thienyl)-thiazol, beschrieben. Die Lösung kann Mannit zur Einstellung der Isotonizität enthalten. Ein Einfluß von Mannit auf den intraokularen Druck ist nicht erwähnt.

Die Publikation Ann. d'Oculist 210(1), S. 63-69 (1977) stellt eine Übersicht über die Anwendung osmoregulatorischer Mittel in der Ophthalmologie vor. Als topisch anzuwendende Mittel werden beispielsweise Glucose und Glycerin zur Behandlung des cornealen Ödems genannt.

In US-4,201,706 ist eine Methode zur Reduktion des cornealen Ödems beschrieben, welche in der topischen Anwendung einer wäßrigen ophthalmischen Lösung, die als Wirkstoff einen Penta- oder Hexahydroxyalkohol wie Sorbit, Inosit oder Xylit enthält, besteht. Das corneale Ödem kann durch ein physikalisches Trauma oder durch Reizeinwirkungen entstehen und wird auch in Assoziation mit Erkrankungen wie dem Glaukom beobachtet.

Es wurde nun gefunden, daß durch die topische Anwendung einer ophthalmischen Lösung, die als Wirkstoff eine therapeutisch wirksame Menge eines Polyhydroxyalkohols ausgewählt aus Sorbit, Mannit, Inosit und Xylit oder deren Mischungen in einem augenverträglichen Träger enthält, der erhöhte intraokulare Druck signifikant gesenkt werden kann, wobei die vorstehend erwähnten systemischen Nebenwirkungen osmoregulatorisch wirkender Verbindungen durch die lokal begrenzte Anwendung und die niedrige Konzentration des Wirkstoffes vermieden werden und infolge des Fehlens vasokonstriktorischer Eigenschaften des Wirkstoffes keine negative Beeinflussung des okularen Blutflusses auftritt.

In einer 28-tägigen klinischen Studie wurde die Wirksamkeit und Verträglichkeit einer 5 % Sorbit enthaltenden wässrigen ophthalmischen Lösung bei 23 Patienten mit erstmalig diagnostiziertem oder bisher unbehandeltem erhöhtem intraokularen Druck überprüft. Das Prüfpräparat wurde jeweils 2mal täglich im Abstand von ca. 12 Stunden angewendet, wobei jeweils 1 Tropfen in jedes Auge gegeben wurde.

### Methodik:

### Ablauf der Prüfung

Im Rahmen der Prüfung fanden 4 Untersuchungstermine statt:
1. Eingangsuntersuchung vor Aufnahme in die Studie
2. Erster Behandlungstag
3. Siebter Behandlungstag
4. Achtundzwanzigster Behandlungstag

An jedem dieser Untersuchungstermine wurde zur Überprüfung der Wirksamkeit ein Tagesprofil des Augeninnendrucks mit Hilfe eines Goldmann-Applanationstonometers erstellt. Die Messungen erfolgten jeweils zu den Stunden 0, 2, 4 und 8 am rechten Auge. Zu den gleichen Zeitpunkten wurde die Pupillengröße des rechten Auges mit einem Millimeterlineal unter Standard-Dämmerbeleuchtung und bei fixiertem Fokus auf ein entferntes Ziel gemessen. Zur Erfassung systemischer Nebenwirkungen erfolgte jeweils die Messung des Blutdrucks und der Herzfrequenz am sitzenden Patienten. Die lokale Verträglichkeit des Präparates wurde durch die subjektive Bewertung des Patienten hinsichtlich des Ausprägungsgrades von Fremdkörpergefühl, Juckreiz und Brennen ermittelt, die objektive okuläre Verträglichkeit wurde anhand einer Spaltlampenuntersucnung von Lid, Bindehaut und Hornhaut am linken Auge beurteilt. Dabei wurde beim Lid und bei der Bindehaut insbesondere auf das Vorhandensein von Erythem und Ödem, bei der Hornhaut auf Ödem und Erosion geachtet.

Im Rahmen der Eingangsuntersuchung wurde eine perimetrische Untersuchung durchgeführt, um eventuelle Gesichtsfeldschäden auszuschließen.

### Ergebnisse:

### Einfluß des Präparates auf den intraokularen Druck:

Bereits nach einem Behandlungstag nahm der intraokulare Druck von einem mittleren Ausgangswert von 27 mm Hg auf einen mittleren Wert von 21 mm Hg ab. Die stärkste Drucksenkung war jeweils 2 Stunden nach der Applikation festzustellen, 8 Stunden nach der Applikation waren die Ausgangswerte noch nicht wieder erreicht. Nach 28 Tagen zeigte sich vor der morgendlichen Applikation eine im Mittel 23%ige, 2 Stunden nach der morgendlichen Applikation eine im Mittel 29%ige Senkung des intraokularen Drucks gegenüber dem ersten Behandlungstag, Stunde O. Am Ende der 28-tägigen Behandlungszeit betrug der intraokulare Druck im Mittelwert 19 mm Hg.

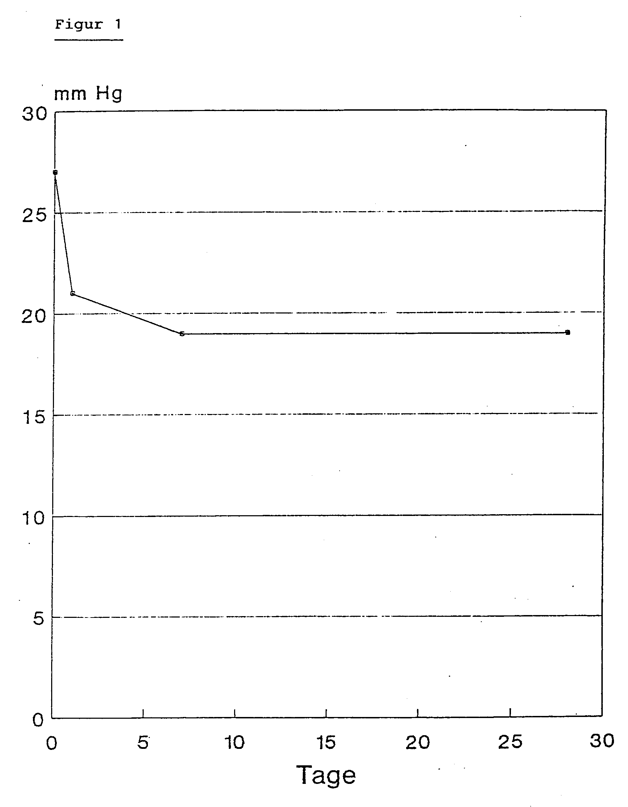

### Legende zu Figur 1:

Die Figur gibt den Verlauf des gemittelten intraokularen Drucks einer Gruppe von 23 Patienten mit intraokularer Hypertension während einer 28-tägigen Behandlung durch topische Applikation einer 5 % Sorbit enthaltenden wässrigen ophthalmischen Lösung wieder. Es wurde jeweils zweimal täglich im Abstand von 12 Stunden ein Tropfen der Lösung in jedes Auge verabreicht. Nach einem signifikanten Absinken des Druckwertes während des ersten Behandlungstages wird ab dem 7. Tag ein konstanter mittlerer Wert von 19 mm Hg erreicht.

### Verträglichkeit und Nebenwirkungen:

Die Messung des Pupillendurchmessers ergab keine Beeinflussung durch die Applikation der ophthalmischen Lösung. Der mittlere Pupillendurchmesser blieb während der gesamten Studiendauer konstant. Bei keinem der Patienten konnte eine Miosis oder Mydriasis festgestellt werden.

Die systolischen und diastolischen Blutdruckwerte sowie die Herzfrequenz wurden durch die Applikation der Augentropfen nicht beeinflußt und es ließen sich auch keine weiteren systemischen Nebenwirkungen beobachten.

Die Ergebnisse der Spaltlampenuntersuchung von Augenlid, Bindehaut und Hornhaut sowie die Befragung der Patienten nach subjektiven Mißempfindungen wie Juckreiz, Brennen oder Fremdkörpergefühl ergab in Zahl und Ausmaß lediglich geringfügige Beeinträchtigungen, die mit anderen, lokal applizierten Ophthalmika vergleichbar sind.

Gegenstand der vorliegenden Erfindung ist somit die Verwendung eines Polyhydroxyalkohols ausgewählt aus Sorbit, Mannit, Inosit oder Xylit zur Herstellung einer ophthalmischen Lösung zur topischen Anwendung am Auge zur Erniedrigung eines erhöhten intraokularen Kammerwasserdrucks,wobei der Polyhydroxyalkohol zweckmäßigerweise in einem augenverträglichen Träger eingearbeitet wird.

In einer bevorzugten Ausführungsform enthält die herzustellende ophthalmische Lösung nur einen der Polyhydroxyalkohole Sorbit, Mannit, Inosit oder Xylit als einzigen Wirkstoff. Besonders bevorzugt ist die Verwendung von Sorbit als einzigem Wirkstoff.

Die Konzentration des Polyhydroxyalkohols in der ophthalmischen Lösung beträgt zwischen 0,5 und 10 Gewichtsprozent, vorzugsweise jedoch 3 bis 7 Gewichtsprozent. Besonders bevorzugt ist eine Konzentration des Wirkstoffes in der ophthalmischen Lösung von 3 bis 5,4 %.

Als augenverträglicher Träger wird beispielsweise eine sterile wässrige Lösung verwendet. Die wäßrige Lösung wird durch Zusatz eines geeigneten Puffers wie z. B. Borsäure, Natriumtetraborat, Natriummonohydrogenphosphat, Natriumdihydrogenphosphat oder Zitronensäure auf einen physiologisch verträglichen pH-Wert von 6 bis 8 eingestellt. Als weitere Bestandteile können der Lösung die dem Fachmann bekannten Hilfsstoffe, z. B. Konservierungsmittel wie Benzalkoniumchlorid, Surfactants, Liposomen oder Polymere wie z. B. Methylcellulose, Polyvinylalkohol oder Polyvinylpyrrolidon zugesetzt werden.

Die ophthalmische Lösung wird zur topischen Anwendung am Auge zur Erniedrigung eines erhöhten intraokularen Kammerwasserdrucks verwendet. Da nach intraokularen Operationen wie z. B. nach Trabekulektomie oder nach Kataractoperationen häufig Störungen der intraokularen Blutwasserschranke auftreten, die eine Erhöhung des intraokularen Kammerwasserdrucks bewirken, eignet sich die ophthalmische Lösung insbesondere auch zur topischen Anwendung am Auge nach intraokularen Operationen.

In der überwiegenden Zahl der Fälle tritt ein erhöhter intraokularer Druck in Assoziation mit dem Glaukom auf. Aus diesem Grund lasssen sich die vorstehend genannten Polyhydroxyalkohole auch in Kombination mit weiteren Arzneimitteln, insbesondere jedoch mit Anti-Glaukom-Mitteln wie z. B. Carboanhydrase-Hemmern, Pilocarpin, α₂-adrenergen Verbindungen wie Clonidin, 2-Amino-6-allyl-4,5,7,8-tetrahydro-6H-thiazolo-[5,4-d]azepin, (-)-2-Amino-6-n-propylamino-4,5,6,7-tetrahydrobenzthiazol und Phenylephrin und seinen Derivaten, zur Herstellung einer ophthalmischen Lösung zur topischen Behandlung des erhöhten intraokularen Kammerwasserdrucks einsetzen.

Die Herstellung der ophthalmischen Lösung erfolgt nach bekannten Methoden durch Vermischen oder Lösen des Wirkstoffes und der üblichen pharmazeutischen Hilfsstoffe in dem augenverträglichen Träger und soll anhand folgender Beispiele erläutert werden, wobei die angegebenen Zusammensetzungen die Erfindung nicht begrenzen sollen.

### Beispiel 1:

### Rezepturen für Sorbit-Augentropfen (Zusammensetzung g/100 ml):

Die angegebenen Lösungen sind, wenn nicht anders angegeben, euhydrisch (pH 6,8-7,1) und eutonisch [ t: -(290-310 mosmol)].

| | | | | | | | | |
|---|---|---|---|---|---|---|---|---|
| Sorbit | 7,00 1) | 5,20 | 3,00 2) | 2,00 3) | 4,75 | 3,00 | 1,00 | 0,60 |
| Kochsalz | | | | | | 0,30 | | |
| Borsäure | | | | 1,25 | | | | |
| Na-tetraborat | | | | 0,15 | | | | |
| Na-hydrogenphosphat | | | | | 0,10 | 0,10 | 1,00 | |
| Na-dihydrogenphosphat | | | | | 0,10 | 0,10 | 1,00 | 2,00 |
| Zitronensäure | | | | | | | | 0,20 |
| Wasser dest. | ad 100 | + | + | + | + | + | + | + |
| Konservans: qu. s. | + | + | + | + | + | + | + | + |

| | | | | | | | | |
|---|---|---|---|---|---|---|---|---|
| 1) hyperton | | | | | | | | |
| 2) hypoton | | | | | | | | |
| 3) schwach sauer | | | | | | | | |

## Patentansprüche

1. Verwendung eines Polyhydroxyalkohols ausgewählt aus Sorbit, Mannit, Inosit oder Xylit oder deren Mischungen als Wirkstoff zur Herstellung einer ophthalmischen Lösung zur topischen Anwendung am Auge zur Erniedrigung eines erhöhten intraokularen Kammerwasserdrucks.

2. Verwendung nach Anspruch 1, dadurch gekennzeichnet, daß die ophthalmische Lösung zusätzlich ein oder mehrere andere Anti-Glaukommittel enthält.

3. Verwendung nach einem der Ansprüche 1 oder 2, dadurch gekennzeichnet, daß der Polyhydroxyalkohol in einer Konzentration zwischen 0,5 und 10 Gewichtsprozent in der ophthalmischen Lösung enthalten ist.

4. Verwendung nach einem der Ansprüche 1 oder 2, dadurch gekennzeichnet, daß der Polyhydroxyalkohol in einer Konzentration von 3 bis 7 Gewichtsprozent in der ophthalmischen Lösung enthalten ist.

5. Verwendung nach einem der Ansprüche 1 oder 2, dadurch gekennzeichnet, daß der Polyhydroxyalkohol in einer Konzentration von 3 bis 5,4 Gewichtsprozent in der ophthalmischen Lösung enthalten ist.

6. Verwendung nach einem der Ansprüche 1 bis 5, dadurch gekennzeichnet, daß der Polyhydroxyalkohol Sorbit ist.

7. Verwendung nach Anspruch 1, dadurch gekennzeichnet, daß der Polyhydroxyalkohol Sorbit ist und in einer Konzentration von 5 Gewichtsprozent in der ophthalmischen Lösung enthalten ist.

8. Verwendung nach einem der Ansprüche 1 bis 7, dadurch gekennzeichnet, daß die ophthalmische Lösung eine wäßrige Lösung ist.

9. Verwendung nach Anspruch 8, dadurch gekennzeichnet, daß die wäßrige Lösung einen pH-Wert zwischen 6 und 8 besitzt und neben einem augenverträglichen Puffer gegebenenfalls ein oder mehrere Konservierungsmittel enthält.

## Claims

1. Use of a polyhydroxy alcohol selected from sorbitol, mannitol, inositol or xylitol or mixtures thereof as active substances for preparing an ophthalmic solution for topical application to the eye in order to reduce elevated intraocular pressure of the aqueous humour.

2. Use according to claim 1, characterised in that the ophthalmic solution additionally contains one or more other anti-glaucoma agents.

3. Use according to one of claims 1 or 2, characterised in that the polyhydroxy alcohol is contained in the ophthalmic solution in a concentration of between 0.5 and 10 percent by weight.

4. Use according to one of claims 1 or 2, characterised in that the polyhydroxy alcohol is contained in the ophthalmic solution in a concentration of from 3 to 7 percent by weight.

5. Use according to one of claims 1 or 2, characterised in that the polyhydroxy alcohol is contained in the ophthalmic solution in a concentration of from 3 to 5.4 percent by weight.

6. Use according to one of claims 1 to 5, characterised in that the polyhydroxy alcohol is sorbitol.

7. Use according to claim 1, characterised in that the polyhydroxy alcohol is sorbitol and is present in the ophthalmic solution in a concentration of 5 percent by weight.

8. Use according to one of claims 1 to 7, characterised in that the ophthalmic solution is an aqueous solution.

9. Use according to claim 8, characterised in that the aqueous solution has a pH of between 6 and 8 and, in addition to a buffer tolerated by the eye, optionally contains one or more preservatives.

## Revendications

1. Utilisation d'un polyhydroxyalcool choisi parmi le sorbitol, le mannitol, l'inositol ou le xylitol ou leurs mélanges comme principe actif pour la préparation d'une solution ophtalmique pour l'application topique sur l'oeil pour abaisser une pression de l'humeur aqueuse intraoculaire accrue.

2. Utilisation selon la revendication 1 caractérisée en ce que la solution ophtalmique contient en outre un ou plusieurs autres agents antiglaucome.

3. Utilisation selon l'une des revendications 1 ou 2 caractérisée en ce que le polyhydroxyalcool est contenu en une concentration comprise entre 0,5 et 10% en masse dans la solution ophtalmique.

4. Utilisation selon l'une des revendications 1 ou 2 caractérisée en ce que le polyhydroxyalcool est contenu en une concentration comprise entre 3 et 7% en masse dans la solution ophtalmique.

5. Utilisation selon l'une des revendications 1 ou 2 caractérisée en ce que le polyhydroxyalcool est contenu en une concentration comprise entre 3 et 5,4% en masse dans la solution ophtalmique.

6. Utilisation selon l'une des revendications 1 à 5 caractérisée en ce que le polyhydroxyalcool est le sorbitol.

7. Utilisation selon la revendication 1 caractérisée en ce que le polyhydroxyalcool est le sorbitol et est contenu en une concentration de 5% en masse dans la solution ophtalmique.

8. Utilisation selon l'une des revendications 1 à 7 caractérisée en ce que la solution ophtalmique est une solution aqueuse.

9. Utilisation selon la revendication 8 caractérisée en ce que la solution aqueuse possède un pH compris entre 6 et 8 et contient éventuellement un ou plusieurs conservateurs outre un tampon acceptable pour l'oeil.
